# EUROPEAN PATENT SPECIFICATION

(11) **EP 1 883 358 B1**
(45) Date of publication and mention of the grant of the patent: **02.12.2009**
(21) Application number: 06726919.1
(22) Date of filing: 26.04.2006
(51) Int. Cl.: A61B 17/28, A61B 17/32, A61B 18/14, B25B 7/00, A61B 17/00

(54) **A SURGICAL INSTRUMENT**
OPERATIONSINSTRUMENT
INSTRUMENT CHIRURGICAL

(30) Priority: 25.05.2005 US 684364 P
(43) Date of publication of application: 06.02.2008
(73) Proprietor: Gyrus Medical, Inc., Maple Grove, MN 55311-3602 (US)
(72) Inventor: BOSSHARD, Mark, Richard, Shoreview, MN 55126 (US); ROBINSON, David, Bryan, Chanhassen, MN 55317 (US)
(74) Representative: Pratt, David Martin
(86) International application number: PCT/GB2006/001535
(87) International publication number: WO 2006/125940

(56) References cited:
- DE-A1- 19 713 067
- DE-U1- 20 000 812
- GB-A- 2 348 390
- US-A- 6 066 159
- US-B1- 6 293 954

## Description

This invention relates to a surgical instrument such as a forceps or scissors device. Such devices have been known for many years, US Patent specification 2,111,161 dating from 1937 describing an early example. The present invention attempts to provide a modern improvement to such devices. US6293954 discloses a surgical clamp having a demi-permanent connection between the jew assembly and the handles, said connection being a sachet connection reinforced by a bonding agent.

The present invention provides a surgical instrument comprising a jaw assembly comprising first and second jaw members pivotally connected one to the other so as to be movable between respective open and closed positions, and first and second handle members connected to the first and second jaw members respectively, the first and second handle members each including a hollow tubular portion at its distal end and having an internal diameter, and the first and second jaw members each including an extension at its proximal end and having an external diameter such that each handle member is solely connected to the associated jaw member by means of a press-fit connection, characterised in that:
the extension at the proximal end of each jaw member has a first end towards the tip thereof, and a second end towards the body of the jaw assembly, and includes one or more externally-facing ribs positioned such that the transverse dimension of each extension is greater at the second end of that extension than at the first end thereof.

The instrument is conveniently an open surgical instrument, that is to say an instrument designed to be used in open surgery. Such an instrument is similar to that described in the US patent specification mentioned above, in that it includes a scissors-like handle with apertures into which the surgeon's fingers can be received. This type of instrument is in contrast to the type used in endoscopic surgery, which typically has actuating elements mounted on a long shaft for insertion into a small aperture in the patient's body.

Typically, the instrument is a forceps instrument, although it is alternatively conceivably a scissors instrument. The instrument is preferably an electrosurgical instrument, with one or more electrodes mounted on the jaw assembly. The press-fit connection is a substantially permanent connection, as opposed to prior art systems such as that described in US Patent specification 6,511,480, which provide removable electrode assemblies. The press-fit connection is sufficiently robust to allow for vigorous use of the surgical instrument, without the concern that the connection may become detached. Thus, the connections of the present invention are "one-time" connections, designed to last for the recommended lifetime of the surgical instrument.

The press-fit connection of the present invention allows for improvements in the cost, weight and component manufacture of the instrument. For example, a single design of handle can be manufactured and connected to different designs of jaw assembly (forceps, scissors etc.), in order to produce a range of instruments. Alternatively, a range of instruments can be provided by means of a single design of jaw assembly, with different designs of handle attached thereto. This may be to accommodate different sizes and shapes for the handles, or merely the preference of one surgeon over another.

According to a preferred arrangement, the hollow tubular portion at the distal end of each handle member is formed of a substantially incompressible material, such as stainless steel. This is in marked contrast to other press-fit connections, which are more generally used with elastomeric materials such as soft plastics and rubber materials. Conveniently, the extension at the proximal end of each jaw member is also formed of a substantially incompressible material, such as stainless steel.

For the purposes of this description, the first end of the extension is the end towards the tip of the extension, and the second end of the extension is the end where the extension is connected to the body of the jaw assembly. These terms are used in so that the terms "proximal" and "distal" can be used in an overall sense with regard to the surgical instrument as a whole, as opposed to locally in respect of a particular component such as the extension.

Preferably, the extension of each jaw member is provided with a plurality of ribs, extending either longitudinally along that extension or radially around that extension. The ribs allow for a secure press-fit connection to be established between the jaw members and the handle member, even when the tubular portions and the extensions are subject to manufacturing tolerances. The ribs can accommodate small variations in the sizes of the two components, without producing an unduly loose press-fit connection, or one which is unduly difficult to assemble.

Where the ribs extend longitudinally along the extensions, each of the ribs has a first height at the first end and a second height at the second end. In this case, the internal diameter of each hollow tubular portion has a first manufacturing tolerance, and the height of the ribs of the associated extension has a second manufacturing tolerance, the height of the ribs being such that, at the Least Material Condition, the internal diameter of that hollow tubular potion is between the first height and the second height. The Least Material Condition is herein defined as being the situation in which the extension on a jaw member is the minimum diameter allowed under the manufacturing tolerance, and the internal diameter of the associated tubular portion is the maximum diameter allowed under the manufacturing tolerance. Thus the Least Material Condition defines the loosest possible fit permissible under the manufacturing tolerances. Even with this loosest fit, the graduation of the heights of the ribs means that one or more of the ribs forms an interference fit with the internal diameter of the associated tubular portion.

Preferably, the height of the ribs of each extension is such that, at the Least Material Condition, the internal diameter of the associated hollow tubular portion is within the middle third of the range between the first height and the second height. For example, if there are nine radially-extending ribs on each extension and a gradual progression in the height of the ribs, the internal diameter of the associated tubular portion will form an interference fit with the fourth to the sixth ribs. Thus, even at Least Material Condition, each tubular portion will form an interference fit with several of the ribs on the associated extension.

Additionally, the height of the ribs of each extension is preferably such that, at the Maximum Material Condition, the internal diameter of the associated hollow tubular portion is greater than or equal to the first height. The Maximum Material Condition is herein defined as being the situation in which the extension on a jaw member is the maximum diameter allowed under the manufacturing tolerance, and the internal diameter of the associated tubular portion is the minimum diameter allowed under the manufacturing tolerance. Thus, the Maximum Material Condition defines the tightest possible fit permissible under the manufacturing tolerances. Even with this tightest fit, the graduation of the heights of the ribs of each extension means that the internal diameter of the associated tubular portion fits over at least one of the ribs to form an interference fit.

As stated above, the height of the ribs of each extension conveniently increases in a steady progression from the first height to the second height. In an alternative arrangement, there can be different groups of ribs, at different heights to other groups of ribs, but with the ribs of each group being all at the same height. In one convenient arrangement, there are one or more additional ribs at the first height towards the first end of each extension. This ensures that, at the Maximum Material Condition, there are several ribs of each extension of equal height in interference fit with the associated tubular portion. Alternatively, or additionally, there are conveniently one or more additional ribs at the second height towards the second end of each extension.

The invention will now be further described, by way of example only, with reference to the accompanying drawings, in which;
Figure 1 is a side view of a forceps instrument constructed in accordance with the present invention;
Figures la and 1b are enlarged sectional views of the circled parts of Figure 1;
Figure 2 is a side view of a jaw member component of the instrument of Figure 1;
Figure 3 is an enlarged view of a portion of the component of Figure 2, showing the ribs thereon; and
Figure 4 is an enlarged view of a portion of the jaw member component of Figure 2, showing an alternative embodiment of the ribs thereon.

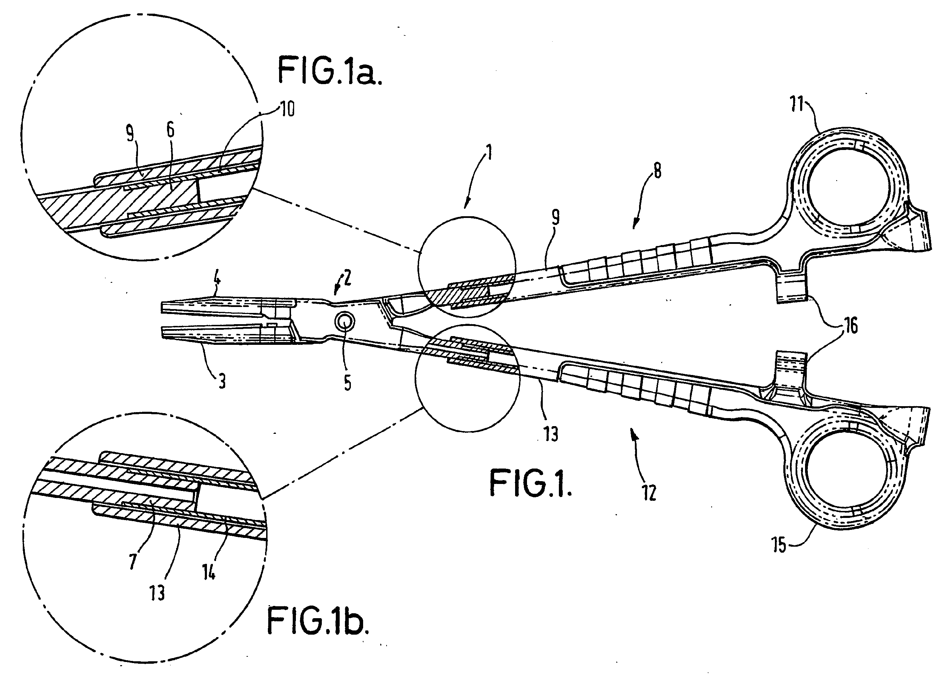

Referring to the drawings, Figure 1 shows an open forceps instrument 1, which comprises a jaw assembly 2 comprising a first jaw member 3 and a second jaw member 4, pivotally connected one to the other by means of a pivot pin 5. The jaw member 3 is provided at its proximal end with an extension 6, and the jaw member 4 is provided with a similar extension 7.

A first handle member 8 is provided, the first handle member comprising a body portion 9, a tubular portion 10 associated with the body portion, and a gripping portion 11 at the proximal end of that handle member. The body portion 9 of the handle member 8 is over-moulded over the tubular portion 10. The handle member 8 is attached to the jaw member 3 by means of the tubular portion 10 forming a press-fit connection with the extension 6. In similar fashion, a second handle member 12 is provided, the second handle member comprising a body portion 13, a tubular portion 14 associated with the body portion, and a gripping portion 15 at the proximal end of that handle member. The handle member 12 is attached to the jaw member 4 by means of the tubular portion 14 forming a press-fit connection with the extension 7.

Movement of the handle members 8 and 12 causes the jaw members 3 and 4 to open and close relative to one another, so that tissue can be gripped therebetween. A ratchet mechanism 16 is provided on each handle member 8, 12 for locking the handle members when they are moved together into their closed position.

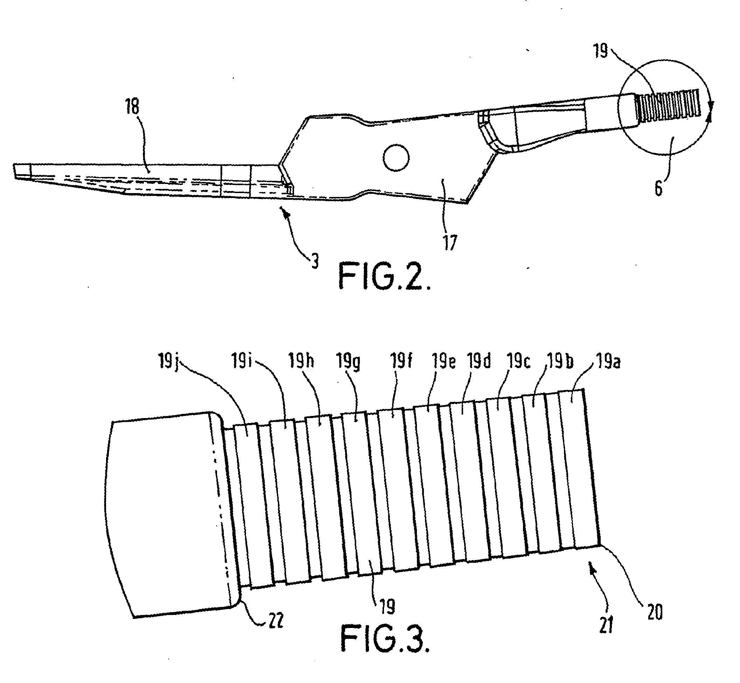

The jaw member 3 is shown in more detail in Figure 2, comprising a central body portion 17, from which depends the extension 6 in one direction and a jaw element 18 in the other direction. The extension 6 is a solid member having a series of ribs 19 provided thereon, the ribs being shown in more detail in Figure 3.

The extension 6 has ten ribs 19a to 19j, extending from a tapered, lead-in portion 20 at a first end 21 of the extension, to the body portion 17 of the jaw member 3 at the second end 22 of the extension. In the example of Figure 3, the ribs 19a to 19j have the following diameters;

| Rib | Diameter |
|---|---|
| 19a | 4.0538 mm (0.1596 inches) |
| 19b | 4.0538 mm (0.1596 inches) |
| 19c | 4.0792 mm (0.1606 inches) |
| 19d | 4.0996 mm (0.1614 inches) |
| 19e | 4.1250 mm (0.1624 inches) |
| 19f | 4.1504 mm (0.1634 inches) |
| 19g | 4.1707 mm (0.1642 inches) |
| 19h | 4.1901 mm (0.1652 inches) |
| 19i | 4.1901 mm (0.1652 inches) |
| 19j | 4.1901 mm (0.1652 inches) |

All dimensions are in mm (inches) and have a tolerance of ± 0.0254 mm (0.001 inches).

The tubular portion 10 of the handle member 8 has an internal diameter of 4.0894 ± 0.0254 mm (0.161 ± 0.001 inches). It can therefore vary between 4.064 and 4.1148 mm (0.0160 and 0.162 inches). At Least Material Condition, the tubular portion 10 will have an internal diameter of 4.1148 mm (0.162 inches), and the ribs 19a to 19j will be the values given above minus 0.0254 mm (0.001 inches). Thus, the tubular portion 10 will pass over the ribs 19a to 19e and form an interference fit with the ribs 19f to 19j. Depending on the compressibility of the material (stainless steel) and the force used by the machinery to apply the handle member 8 to the jaw member 3, the tubular portion 10 will be engaged with some or all of ribs 19f to 19j. With automated machinery for applying the handle member 8, the tubular portion 10 can be applied over the ribs 19f to 19j without causing the splitting of the tubular portion. There will, therefore, be five ribs (19f to 19j) securing the extension 6 within the tubular portion 10.

At Maximum Material Condition the tubular portion 10 will have an internal diameter of 4.064 mm (0.160 inches), and the ribs 19a to 19j will be the values given above plus 0.0254 mm (0.001 inches). Thus, the tubular portion 10 will form an interference fit with all of the ribs 19a to 19j. There will, therefore, be ten ribs (19a to 19j) securing the extension 6 within the tubular portion 10. Although not described, it will be appreciated that the handle member 12 is secured to the extension of the jaw member 4 in the same manner.

As will be seen from the above description, the provision of the ribs 19a to 19j allows for a secure press-fit connection between the handle members 8 and 12 and the jaw members 3 and 4, despite the tolerances permitted for the manufactured components. This can allow the components to be manufactured by moulding, with a conventional tolerance of ± 00254 mm (± 0.001 inches), as opposed to being manufactured by precision grinding with a much tighter tolerance.

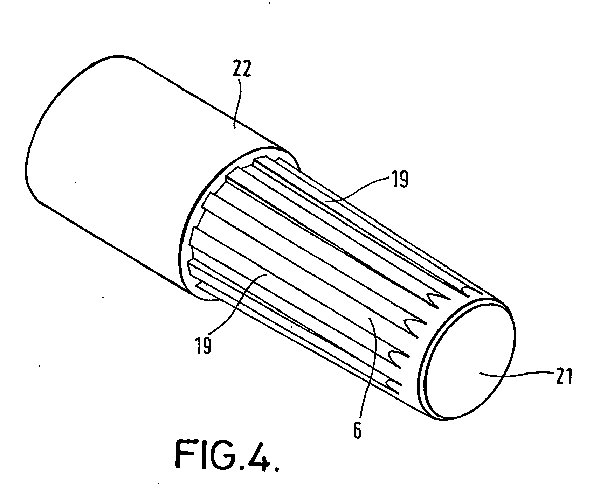

Figure 4 shows an alternative arrangement, in which the ribs 19a to 19j extend longitudinally along the extension 6. The ribs 19a to 19j slope such that they are at a first, relatively shallow height towards the first end 21 of the extension, and are at a second, more pronounced height towards the second end 22 of the extension. The heights of the ribs 19a to 19j are such that, at the Least Material Condition, the internal diameter of the hollow tubular portion 10 is within the middle third of the ribs 19a to 19j. Thus, when the tubular portion 10 is at its loosest, it still engages with a substantial axial length of the longitudinal ribs 19a to 19j. Similarly, the height of the ribs 19a to 19j is such that, at the Maximum Material Condition, the internal diameter of the hollow tubular portion 10 is greater than or equal to the first height. Thus, when the tubular portion 10 is at its tightest, it still fits over the shallowest part of the ribs 19a to 19j, so as to be engaged within their longitudinal extent.

## Claims

1. A surgical instrument (1) comprising a jaw assembly (2) comprising first and second jaw members (3, 4) pivotally connected one to the other so as to be movable between respective open and closed positions, and first and second handle members (8, 12) connected to the first and second jaw members respectively, the first and second handle members each including a hollow tubular portion (10, 14) at its distal end and having an internal diameter, and the first and second jaw members each including an extension (6, 7) at its proximal end and having an external diameter such that each handle member is solely connected to the associated jaw member by means of a press-fit connection, wherein the extension at the proximal end of each jaw member has a first end (21) towards the tip thereof, and a second end (22) towards the body of the jaw assembly, **characterised in that** the extension mcludes one or more externally-facing ribs (19a, 19j) positioned such that the transverse dimension of each extension is greater at the second end of that extension than at the first end thereof, wherein the extension of each jaw member is provided a plurality of ribs (19a to 19j), and wherein the ribs of each extension extend longitudinally along that extension, each of the ribs having a first height at the first end (21) and a second height at the second end (22), wherein the internal diameter of each hollow tubular portion has a first manufacturing tolerance, and the height of the ribs of the associated extension has a second manufacturing tolerance, the height of the ribs being such that, at the Least Material Condition, namely the loosest possible fit permissible under the manufacturing tolerances, the internal diameter of that hollow tubular portion is between the first height and the second height.

2. A surgical instrument according to claim 1, wherein the instrument (1) is an open surgical instrument.

3. A surgical instrument according to claim 1, wherein the instrument (1) is a forceps instrument.

4. A surgical instrument according to claim 1, wherein the instrument (1) is a scissors instrument.

5. A surgical instrument according to any one of claims 1 to 4, wherein the instrument (1) is an electrosurgical instrument.

6. A surgical instrument according to any one of claims 1 to 5, wherein the hollow tubular portion (10, 14) at the distal end of each handle member (18, 12) is formed of a substantially incompressible material.

7. A surgical instrument according to any one of claims 1 to 6, wherein the extension (6, 7) at the proximal end of each jaw member (3, 4) is formed of a substantially incompressible material.

8. A surgical instrument according to any one of claims 1 to 7, wherein the height of the ribs (19a to 19j) of each extension (6, 7) is such that, at the Least Material Condition, the internal diameter of the associated hollow tubular portion (10, 14) is within the middle third of the range between the first height and the second height.

9. a surgical instrument according to any one of claims 1 to 8, wherein the height of the ribs (19a to 19j) of each extension (6, 7) is such that, at the Maximum Material Condition, namely the tightest possible fit permissible under the manufacturing tolerances, the internal diameter of the associated hollow tubular portion (10, 14) is greater than or equal to the first height.

10. A surgical instrument according to any one of claims 1 to 9 wherein the height of the ribs (19a to 19j) of each extension (6, 7) increases in a steady progression from the first height to the second height.

11. A surgical instrument (1) comprising a jaw assembly (2) comprising first and second jaw members (3, 4) pivotally connected one to the other so as to be movable between respective open and closed positions, and first and second handle members (8, 12) connected to the first and second jaw members respectively, the first and second handle members each including a hollow tubular portion (10, 14) at its distal end and having an internal diameter, and the first and second jaw members each including an extension (6, 7) at its proximal end and having an external diameter such that each handle member is solely connected to the associated jaw member by means of a press-fit connection, wherein the extension at the proximal end of each jaw member has a first end (21) towards the tip thereof, and a second end (22) towards the body of the jaw assembly **characterised in that** the extension includes one or more externally-facing ribs (19a, 19j) positioned such that the transverse dimension of each extension is greater at the second end of that extension than at the first end thereof, wherein the extension (6, 7) of each jaw member (3, 4) is provided a plurality of ribs (19a to 19j), and wherein the ribs of each extension extend radially around that extension, the rib (19a) at the first end (21) having a first height, and the rib (19j) at the second end (22) having a second height,
wherein the internal diameter of each hollow tubular portion has a first manufacturing tolerance, and the height of the ribs of the associated extension has a second manufacturing tolerance, the height of the ribs being such that, at the Least Material Condition, the internal diameter of that hollow tubular portion is between the first height and the second height.

12. A surgical instrument according to claim 11, wherein the instrument (1) is an open surgical instrument.

13. A surgical instrument according to claim 11, wherein the instrument (1) is a forceps instrument.

14. A surgical instrument according to claim 11, wherein the instrument (1) is a scissors instrument.

15. A surgical instrument according to any one of claims 11 to 14, wherein the instrument (1) is an electrosurgical instrument.

16. A surgical instrument according to any one of claims 11 to 15, wherein the hollow tubular portion (10, 14) at the distal end of each handle member (18, 12) is formed of a substantially incompressible material.

17. A surgical instrument according to any one of claims 11 to 16, wherein the extension (6, 7) at the proximal end of each jaw member (3, 4) is formed of a substantially incompressible material.

18. A surgical instrument according to any one of claims 11 to 17, wherein the height of the ribs (19a to 19j) of each extension (6, 7) is such that, at the Least Material Condition, the internal diameter of the associated hollow tubular portion (10, 14) is within the middle third of the range between the first height and the second height.

19. A surgical instrument according to any one of claims 11 to 18, wherein the height of the ribs (19a to 19j) of each extension (6, 7) is such that, at the Maximum Material Condition, the internal diameter of the associated hollow tubular portion (10, 14) is greater than or equal to the first height.

20. A surgical instrument according to any one of claims 11 to 19, wherein the height of the ribs (19a to 19j) of each extension increases in a steady progression from the first height to the second height.

21. A surgical instrument according to any one of claims 11 to 20, wherein there are one or more additional ribs at the first height towards the first end (21) of each extension (6, 7).

22. A surgical instrument according to anyone of claims 11 to 21, wherein there are one or more additional ribs at the second height towards the second end (22) of each extension (6, 7).

## Patentansprüche

1. Chirurgisches Instrument (1), umfassend eine βackenanordnung (2) mit einem ersten und einem zweiten Backenelement (3,4), die schwenkbar miteinander verbunden sind, so dass sie jeweils zwischen einer offenen und geschlossenen Position bewegbar sind, und ein erstes und ein zweites Griffelement (8, 12), die jeweils mit dem ersten und dem zweiten Backenelement verbunden sind, wobei das erste und das zweite Griiffelement an ihrem distalen Ende jeweils einen hohlen rohrförmige Abschnitt (10,14) und einen Innendurchmesser aufweisen, und wobei das erste und das zweiten Backenelement an ihrem proximalen Ende jeweils eine Verlängerung (6, 7) und einen Außendurchmesser derart haben, dass jedes Griffelement durch eine Presssitzverbindung einzig mit dem zugeordneten Backenelement verbunden ist, wobei die Verlängerung an dem proximalen Ende jedes Backenelements ein erstes Ende (21) in Richtung auf seine Spitze und ein zweites Ende (22) in Richtung auf den Körper der Backenanordnung aufweist, **dadurch gekennzeichnet, dass** die Verlängerung eine oder mehrere nach außer weisende Rippen (19a, 19j) aufweist, die so positioniert sind, dass die Querabmessung jeder Verlängerung an dem zweiten Ende dieser Verlängerung größer ist als an dem ersten Ende, wobei die Verlängerung jedes Backenelements mit einer Vielzahl von Rippen (19a bis 19j) versehen ist und wobei die Rippen jeder Verlängerung sich entlang dieser Verlängerung in Längsrichtung erstrecken, wobei jede der Rippen an dem ersten Ende (21) eine erste Höhe und an dem zweiten Ende (22) eine zweite Höhe hat, wobei der Innendurchmesser jedes hohlen rohrförmigen Abschnitts eine erste Herstellungstoleranz und die Höhe der Rippen der zugehörigen Verlängerung eine zweite Herstellungstoleranz aufweist, wobei die Höhe der Rippen dergestalt ist, dass unter der Minimum-Material-Bedingung, nämlich der unter den Herstellungstoleranzen möglichen lockersten Passung, der Innendurchmesser des hohlen rohrförmigen Bereichs zwischen der ersten und der zweiten Höhe liege.

2. Chirurgisches Instrument nach Anspruch 1, wobei das Instrument (1) ein offenes chirurgisches instrument ist.

3. Chirurgisches Instrument nach Anspruch 1, wobei das Instrument (1) ein Zangen instrument ist.

4. Chirurgisches Instrument nach Anspruch 1, wobei das Instrument (1) ein Schereninstrument ist.

5. Chirurgisches Instrument nach einem der Ansprüche 1 bis 4, wobei das Instrument (1) ein elektrochirurgisches Instrument ist.

6. Chirurgisches Instrument nach einem der Ansprüche 1 bis 5, wobei der hohle rohrförmige Abschnitt (10,14) an dem distalen Ende jedes Griffelements (18, 2) aus einem im Wesentlichen inkompressiblen Material gebildet ist.

7. Chirurgisches Instrument nach einem der Ansprüche 1 bis 6, wobei die Verlängerung (6, 7) an dem proximalen Ende jedes Backenelements (3, 4) aus einem im Wesentlichen inkompressiblen Material gebildet ist.

8. Chirurgisches Instrument nach einem der Ansprüche 1 bis 7, wobei die Höhe der Rippen (19a bis 19j) jeder Verlängerung (6, 7) derart ist, dass unter der Minimum-Material-Bedingung der Innendurchmesser des zugeordneten hohlen rohrförmigen Abschnitts (10, 14) in dem mittleren Drittel des Bereichs zwischen der ersten und der zweiten Höhe liegt..

9. Chirurgisches Instrument nach einem der Ansprüche 1 bis 8, wobei die Höhe der Rippen (19a bis 19j) jeder Verlängerung (6, 7) derart ist, dass unter der Maximum-Material-Bedingung, nämlich dem unter den Herstetfungstoteranzen engsten möglichen Sitz, der Innendurchmesser des zugeordneten hohlen rohrförmigen Abschnitts (10, 14) größer oder gleich der ersten Höhe ist.

10. Chirurgisches Instrument nach einem der Ansprüche 1 bis 9, wobei die Höhe der Rippen (19a bis 19j) jeder Verlängerung (6, 7) in einem stetigen Fortschritt von der ersten bis zur zweiten Höhe zunimmt.

11. Chirurgisches (instrument (1), umfassend eine Backenanordnung (2) mit einem ersten und einem zweiten Backenelement (3,4), die schwenkbar miteinander verbunden sind, so dass sie jeweils zwischen einer offenen und geschlossenen Position bewegbar sind, und ein erstes und ein zweites Griffelement (8, 12), die jeweils mit dem ersten und dem zweiten Backenelement verbunden sind, wobei das erste und das zweites Griffelement an ihrem distalen Ende jeweils einen hohlen rohrförmigen Abschnitt (10,14) und einen Innendurchmesser aufweisen, und wobei das erste und das zweiten Backenelement an ihrem proximalen Ende jeweils eine Verlägerung (6, 7) und einen Außendurchmesser derart haben, dass jedes Griffelement durch eine Presssitzverbindung einzig mit dem zugeordneten Backenelement verbunden ist, wobei die Verlängerung an dem proximalen Ende jedes Backenelements ein erstes Ende (21) in Richtung auf seine Spitze und ein zweites Ende (22) in Richtung auf den Körper der Backenanordnung aufweist, **dadurch** gekenntzeichnet, dass die Verlängerung eine oder mehrere nach außen weisende Rippen (1 9a, 19j) aufweist, die so positioniert sind, dass die Querabmessung jeder Verlängerung an dem zweiten Ende dieser Verlängerung größer ist als an dem ersten Ende, wobei die Verlängerung (6, 7) jedes Backenetements (3, 4) mit einer Vielzahl von Rippen (19a bis 19j) versehen ist und wobei die Rippen jeder Verlängerung sich radial um diese Verlängerung verstrecken, wobei die Rippe (19a) an dem ersten Ende (21) eine erste Höhe und an dem zweiten Ende (22) eine zweite Höhe und die Rippe (19j) an dem zweiten Ende (22) eine zweite Höhe hat, wobei der Innendurchmesser jedes hohlen rohrförmigen Abschnitts eine erste Herstellungstoleranz und die Höhe der Rippen der zugehörigen Verlängerung eine zweite Herstellungstoleranz aufweist, wobei die Höhe der Rippen dergestalt ist, dass unter der Minimum-Material-Bedingung, nämlich der unter den Herstellungstoleranzen lockert möglichen Passung, der Innendurchmesser des hohlen rohrförmigen Bereichs zwischen der ersten und der zweiten Höhe liegt.

12. Chirurgisches Instrument nach Anspruch 11, wobei das Instrument (1) ein offenes chirurgisches Instrument ist.

13. Chirurgisches Instrument nach Anspruch 1, wobei das Instrument (1) ein Zangeninstrument ist.

14. Chirurgisches Instrument nach Anspruch 1, wobei das Instrument (1) ein Schereninstrument ist.

15. Chirurgisches Instrument nach einem der Ansprüche 11 bis 14, wobei das Instrument (1) ein (elctrochirurgisches Instrument ist.

16. Chirurgisches Instrument nach einem der Ansprüche 11 bis 15, wobei der hohle rohrförmige Abschnitt (10,14) an dem distalen Ende jedes Griffelementes (18, 12) aus einem im Wesentlichen inkompressiblen Material gebildet ist.

17. Chirurgisches Instrument nach einem der Ansprüche 11 bis 16, wobei die Verlängerung (6,7) an dem proximalen Ende jedes Backenelements (3, 4) aus einem im Wesentlichen inkompressiblen Material gebildet ist.

18. Chirurgisches Instrument nach einem der Ansprüche 11 bis 17, wobei die Höhe der Rippen (19a bis 19j) jeder Verlängerung (6, 7) derart ist, dass unter der Minimum-Material-Bedingung der Innendurchmesser des zugeordneten hohlen rohrförmigen Abschnitts (10,14) in dem mittleren Drittel des Bereichs zwischen der ersten und der zweiten Höhe liegt.

19. Chirurgisches Instrument nach einem der Ansprüche 11 bis 18, wobei die Höhe der Rippen (19a bis 19j) jeder Verlängerung (6, 7) derart ist, dass unter der Maximum-Materiat-Bedingung, nämlich dem unter den Herstellungstoleranzen möglichen engsten Sitz, der innendurchmesser des zugeordneten hohlen rohrförmigen Abschnitts (10, 14) größer oder gleich der ersten Höhe ist.

20. Chirurgisches Instrument nach einem der Ansprüche 11 bis 19, wobei die Höhe der Rippen (19a bis 19j) jeder Verlängerung in einem stetigen Fortschritt von der ersten bis zur zweiten Höhe zunimmt.

21. Chirurgisches Instrument nach einem der Ansprüche 1 bis 20, wobei eine oder mehrere zusätzliche Rippen an der ersten Höhe in Richtung auf das erste Ende (21) jeder Verlängerung (6, 7) vorhanden sind.

22. Chirurgisches Instrument nach einem der Ansprüche 11 bis 21, wobei eine oder mehrere zusätzliche Rippen an der zweiten Höhe in Richtung auf das zweite Ende (22) jeder Verlängerung (6, 7) vorhanden sind.

## Revendications

1. Instrument chirurgical (1) comprenant un ensemble formant une mâchoire (2) comprenant des premier et deuxième éléments de mâchoire (3, 4) reliés l'un à l'autre de façon à pivoter, de sorte qu'ils puissent bouger entre des positions ouverte et fermée respectives, et des premier et deuxième éléments de poignée (8, 12) raccordés aux premier et deuxième éléments de mâchoire, respectivement, les premier et deuxième éléments de poignée comprenant respectivement une partie tubulaire creuse (10, 14) au niveau de son extrémité distale et ayant un diamètre interne, et les premier et deuxième éléments de mâchoire comprenant respectivement un prolongement (6, 7) au niveau de son extrémité proximale et ayant un diamètre externe tel que chaque élément de poignée soit raccordé uniquement à l'élément de mâchoire associé par un moyen de raccordement par emboîtement, le prolongement au niveau de l'extrémité proximale de chaque élément de mâchoire ayant une première extrémité (21) s'étendant vers la pointe de celui-ci, et une deuxième extrémité (22) vers le corps de l'ensemble formant une mâchoire ; **caractérisé en ce que** le prolongement comprend une ou plusieurs nervures tournées vers l'extérieur (19a, 19j) placées de telle sorte que la dimension transversale de chaque prolongement soit supérieure au niveau de la deuxième extrémité de ce prolongement par rapport à la première extrémité de celui-ci, le prolongement de chacun des éléments de mâchoire étant doté d'une pluralité de nervures (19a à 19j) et les nervures de chaque prolongement s'étendant longitudinalement le long de ce prolongement, chacune des nervures ayant une première hauteur au niveau de la première extrémité (21) et une deuxième hauteur au niveau de la deuxième extrémité (22), le diamètre interne de chaque partie tubulaire creuse ayant une première tolérance de fabrication, et la hauteur des nervures du prolongement associé ayant une deuxième tolérance de fabrication, la hauteur des nervures étant telle que, dans les moindres conditions de matériau, à savoir, l'ajustement le plus lâche possible autorisé selon les tolérances de fabrication, le diamètre interne de cette partie tubulaire creuse se situe entre la première hauteur et la deuxième hauteur.

2. Instrument chirurgical selon la revendication 1, l'instrument (1) étant un instrument chirurgical ouvert.

3. Instrument chirurgical selon la revendication 1, l'instrument (1) étant un instrument de type pince.

4. Instrument selon la revendication 1, l'instrument (1) étant un instrument de type ciseau.

5. Instrument chirurgical selon l'une quelconque des revendications 1 à 4, l'instrument (1) étant un instrument électrochirurgical.

6. Instrument chirurgical selon l'une quelconque des revendications 1 à 5, dans lequel la partie tubulaire creuse (10, 14) au niveau de l'extrémité distale de chacun des éléments de poignée (18, 12) est formée d'un matériau substantiellement incompressible.

7. Instrument chirurgical selon l'une quelconque des revendications 1 à 6, dans lequel le prolongement (6, 7) au niveau de l'extrémité proximale de chaque élément de mâchoire (3, 4) est formé d'un matériau substantiellement incompressible.

8. Instrument chirurgical selon l'une quelconque des revendications 1 à 7, dans lequel la hauteur des nervures (19a à 19j) de chaque prolongement (6, 7) est telle que, dans les moindres conditions de matériau, le diamètre interne de la partie tubulaire creuse associée (10, 14) soit dans le tiers médian de l'ordre de grandeur compris entre la première hauteur et la deuxième hauteur.

9. Instrument chirurgical selon l'une quelconque des revendications 1 à 8, dans lequel la hauteur des nervures (19a à 19j) de chaque prolongement (6, 7) est telle que dans les conditions maximales de matériau, à savoir l'ajustement le plus étroit possible autorisé selon les tolérances de fabrication, le diamètre interne de la partie tubulaire creuse associée (10, 14) soit supérieur ou égal à la première hauteur.

10. Instrument chirurgical selon l'une quelconque des revendications 1 à 9, dans lequel la hauteur des nervures (19a à 19j) de chaque prolongement (6, 7) augmente en progression constante de la première hauteur à la deuxième hauteur.

11. Instrument chirurgical (1) comprenant un ensemble formant une mâchoire (2) comprenant des premier et un deuxième éléments de mâchoire (3, 4) reliés l'un à l'autre de façon à pivoter, de sorte qu'ils puissent bouger entre des positions ouverte et fermée respectives, et des premier et deuxième éléments de poignée (8, 12) raccordés aux premier et deuxième éléments de mâchoire, respectivement, les premier et deuxième éléments de poignée comprenant respectivement une partie tubulaire creuse (10, 14) au niveau de son extrémité distale et ayant un diamètre interne, et les premier et deuxième éléments de mâchoire comprenant respectivement un prolongement (6, 7) au niveau de son extrémité proximale et ayant un diamètre externe tel que chaque élément de poignée soit raccordé uniquement à l'élément de mâchoire associé par un moyen de raccordement par emboîtement, le prolongement au niveau de l'extrémité proximale de chaque élément de mâchoire ayant une première extrémité (21) s'étendant vers la pointe de celui-ci, et une deuxième extrémité (22) vers le corps de l'ensemble formant une mâchoire ; **caractérisé en ce que** le prolongement comprend une ou plusieurs nervures tournées vers l'extérieur (19a, 19j) placées de telle sorte que la dimension transversale de chaque prolongement soit supérieure au niveau de la deuxième extrémité de ce prolongement par rapport à la première extrémité de celui-ci, le prolongement (6, 7) de chacun des éléments de mâchoire (3, 4) étant doté d'une pluralité de nervures (19a à 19j) et les nervures de chaque prolongement s'étendant radialement autour de ce prolongement, la nervure (19a) au niveau de la première extrémité (21) ayant une première hauteur et la nervure (19j) au niveau de la deuxième extrémité (22) ayant une deuxième hauteur, le diamètre interne de chaque partie tubulaire creuse ayant une première tolérance de fabrication, et la hauteur des nervures du prolongement associé ayant une deuxième tolérance de fabrication, la hauteur des nervures étant telle que, dans les moindres conditions de matériau, le diamètre interne de cette partie tubulaire creuse se situe entre la première hauteur et la deuxième hauteur.

12. Instrument chirurgical selon la revendication 11, l'instrument (1) étant un instrument chirurgical ouvert.

13. Instrument chirurgical selon la revendication 11, l'instrument (1) étant un instrument de type pince.

14. Instrument selon la revendication 11, l'instrument (1) étant un instrument de type ciseau.

15. Instrument chirurgical selon l'une quelconque des revendications 11 à 14, l'instrument (1) étant un instrument électrochirurgical.

16. Instrument chirurgical selon l'une quelconque des revendications 11 à 15, dans lequel la partie tubulaire creuse (10, 14) au niveau de l'extrémité distale de chacun des éléments de poignée (18, 12) est formée d'un matériau substantiellement incompressible.

17. Instrument chirurgical selon l'une quelconque des revendications 11 à 16, dans lequel le prolongement (6, 7) au niveau de l'extrémité proximale de chaque élément de mâchoire (3, 4) est formé d'un matériau substantiellement incompressible.

18. Instrument chirurgical selon l'une quelconque des revendications 11 à 17, dans lequel la hauteur des nervures (19a à 19j) de chaque prolongement (6, 7) est telle que, dans les moindres conditions de matériau, le diamètre interne de la partie tubulaire creuse associée (10, 14) soit dans le tiers médian de l'ordre de grandeur compris entre la première hauteur et la deuxième hauteur.

19. Instrument chirurgical selon l'une quelconque des revendications 11 à 18, dans lequel la hauteur des nervures (19a à 19j) de chaque prolongement (6, 7) est telle que dans les conditions maximales de matériau, le diamètre interne de la partie tubulaire creuse associée (10, 14) soit supérieur ou égal à la première hauteur.

20. Instrument chirurgical selon l'une quelconque des revendications 11 à 19, dans lequel la hauteur des nervures (19a à 19j) de chaque prolongement augmente en progression constante de la première hauteur à la deuxième hauteur.

21. Instrument chirurgical selon l'une quelconque des revendications 11 à 20, dans lequel se trouvent une ou plusieurs nervures supplémentaires au niveau de la première hauteur vers la première extrémité (21) de chaque prolongement (6, 7).

22. Instrument chirurgical selon l'une quelconque des revendications 11 à 21, dans lequel se trouvent une ou plusieurs nervures au niveau de la deuxième hauteur vers la deuxième extrémité (22) de chaque prolongement (6, 7).
